# EUROPEAN PATENT SPECIFICATION

(11) **EP 1 894 543 B1**
(45) Date of publication and mention of the grant of the patent: **21.03.2012**
(21) Application number: 07016426.4
(22) Date of filing: 22.08.2007
(51) Int. Cl.: A61F 2/01

(54) **Vein filter**
Filter für die Vene
Filtre pour veine

(30) Priority: 29.08.2006 US 840888 P
(43) Date of publication of application: 05.03.2008
(60) Divisional application: 10178672.1
(73) Proprietor: Rex Medical, L.P., Conshohocken, PA 19428 (US)
(72) Inventor: McGuckin, James F. Jr., Radnor, PA 19087 (US); Bressler, James E., Langhorne, PA 19047 (US); Schaller, David M., Wallingford, PA 19086 (US)
(74) Representative: Jackson, Derek Charles

(56) References cited:
- EP-A1- 1 616 530
- WO-A-01/10342
- WO-A-2005/117750
- WO-A-2006/036457
- DE-A1- 3 429 850
- US-A1- 2006 079 928
- US-A1- 2006 106 417

## Description

### BACKGROUND

### Technical Field

This application relates to a vascular filter and more particularly to a vein filter for capturing blood clots within the vessel.

### Background of Related Art

Passage of blood clots to the lungs is known as pulmonary embolism. These clots typically originate in the veins of the lower limbs and can migrate through the vascular system to the lungs where they can obstruct blood flow and therefore interfere with oxygenation of the blood. Pulmonary embolisms can also cause shock and even death.

In some instances, blood thinning medication, e.g. anticoagulants such as Heparin, or sodium warfarin can be given to the patient. These medications, however, have limited use since they may not be able to be administered to patients after surgery or stroke or given to patients with high risk of internal bleeding. Also, this medication approach is not always effective in preventing recurring blood clots.

Therefore, surgical methods to reduce the likelihood of such pulmonary embolisms by actually blocking the blood clot from reaching the lungs have been developed. To this end, minimally invasive surgical techniques have been developed involving the placement of a mechanical barrier in the inferior vena cava. These barriers are in the form of filters and are typically inserted through either the femoral vein in the patient's leg or the right jugular vein in the patient's neck or arm under local anesthesia. The filters are then advanced intravascularly to the inferior vena cava where they are expanded to block migration of the blood clots from the lower portion of the body to the heart and lungs.

These prior filters take various forms. One type of filter is composed of coiled wires such as disclosed in U.S. Patent Nos. 5,893,869 and 6,059,825. Another type of filter consists of legs with free ends having anchors for embedding in the vessel wall to hold the filter. These filters are disclosed, for example, in U.S. Patent Nos. 4,688,553, 4,781,173, 4,832,055, and 5,059,205, 5,984,947 and 6,007,558. Another type of filter is disclosed in U.S. Patent no. 6,214,025 consisting of wires twisted together to form a cylindrical anchoring portion conforming to the inner vessel wall surface to exert a radial force and a conical filtering portion.

Several factors have to be considered in designing vein filters. One factor is that the filter needs to be securely anchored within the vessel wall, while avoiding traumatic engagement and damage to the wall as well as damage to the neighboring abdominal aorta. Another factor is that the filter must be collapsible to a sufficiently small size to be easily maneuvered and atraumatically advanced intravascularly to the inferior vena cava or other target vessel. Thirdly, the filter should direct the blood clots to the center of the vessel to improve dissolution of the clot within the vessel by the blood flow.

The filters disclosed in the commonly assigned co-pending application 10/889,429 (hereinafter "the '429 application") (EP-A-1 616 530), satisfy the foregoing parameters. The filters have sufficient anchoring force to retain the filter within the vessel while providing atraumatic contact with the vessel wall, have a minimized insertion (collapsed) profile to facilitate delivery through the vascular system to the surgical site, and direct migration of the captured blood clots to the center of the vessel. The filters also provide simplified insertion through the femoral or the right jugular vein or arm into the inferior vena cava.

The filters of the '429 application can advantageously be readily removed minimally invasively, e.g. intravascularly, from the patient, thus advantageously providing for a temporary filter. Thus, these filters advantageously strike the balance of having structure to provide sufficient anchoring while enabling atraumatic removal from the vessel after a period of time. Certain filters of the '429 application also advantageously have a retrieval end configured to facilitate grasping by a snare as well as to facilitate withdrawal by providing a smooth transition into a retrieval sheath.

The filters of the '429 are very effective in achieving their desired functions, whether used as a permanent or temporary filter. It is an object of the present application to provide a modification to the filters to even further facilitate removal if used as a temporary filter.

The filters of the '429 application also have effective retention hooks to grasp the vessel wall to prevent migration of the filter. It is an object of the present application to provide an alternative retention hook to even further enhance retention.

WO2006/036457 describes a vessel filter having a first region having a first set of struts forming a mounting portion and a filter portion having a converging region at a first portion to direct particles towards the center of the filter. The mounting portion is flared in the expanded position to have a transverse dimension increasing toward a second portion opposite the first portion. A second set of struts forms a second mounting portion flared in the expanded position. A plurality of spaced apart struts extend between the first and second regions. The second struts may form a second filter portion.

US-A-2006/079928 describes a permanent blood clot filter which may be retrieved. The blood clot filter includes a filter section and an alignment section. The filter section includes a filter hub and a set of filter legs whose downstream ends are connected to the filter hub. The filter legs extend axially and radially outwardly from the filter hub to form a conical configuration. The alignment section includes an alignment hub and a set of alignment ribs whose downstream ends are connected to the alignment hub and whose upstream ends are connected to the filter legs. The alignment ribs extend from the alignment hub radially outwardly and then further extend radially inwardly to provide centering of the filter.

### SUMMARY

According to the present invention there is provided a vessel filter comprising a first region and a second region, the filter movable between a collapsed position for delivery to the vessel and a range of expanded positions for placement within the vessel, the first region having a filter portion having a converging region to direct particles toward the center of the filter, the first region including a plurality of spaced apart elongated struts and a plurality of connecting struts extending at an angle from the elongated struts, the second region being flared in the expanded positions to have a transverse dimension increasing toward a second end portion opposite the filter portion, the second region including a vessel engaging portion at the second end portion having a plurality of vessel engaging hooks, the region containing the vessel engaging hooks having a first transverse dimension, the first region having a spacer extending radially with respect to a longitudinal axis of the filter, the spacer having a second transverse dimension, wherein in an unconstrained expanded position the second transverse dimension of the spacer is less than the first transverse dimension of the vessel engaging hook region to provide a lower profile at the spacer region, and wherein the spacer is formed from a spiral cutout in the first region of the filter.

In one embodiment, the spacer comprises two portions extending on opposite sides of the filter, for example as two looped portions.

The spacer may be formed integrally with the filter and may be formed of shape memory material.

In some embodiments, two spacers may be provided.

The vessel engaging portion may include vessel engaging hooks having a plurality of teeth. The vessel engaging hooks may include a heel extending past the hook.

In one embodiment, the filter is formed from a laser cut tube composed of shape memory material.

In one embodiment, the converging region terminates in a tubular portion, each of the elongated struts in the first region extends outwardly from the tubular portion, the at least one spacer in the expanded position extending radially from the tubular portion. The spacer may be formed from a spiral cut in the tubular portion. The spacer may have a looped shape memory position and during delivery may have a collapsed position substantially flush (aligned) with the tubular portion.

In one embodiment the first region may further include a retrieval region, the retrieval region including a hook having a cutout exposing an internal annular surface, the annular surface dimensioned to receive a portion of a retrieval sheath.

### BRIEF DESCRIPTION OF THE DRAWINGS

Preferred embodiment(s) of the present disclosure are described herein with reference to the drawings wherein:
Figure 1 is a perspective view of a first embodiment of a vein filter of the present invention in the collapsed (retracted) configuration, and shown removed from a delivery tube/sheath;
Figure 2 is an enlarged broken side view of a portion of the vein filter of Figure l;
Figure 3 is a developed view of the retention hooks of the vein filter of Fig. 1;
Figure 4A is a perspective view of the vein filter of Figure 1 in an expanded (radially extending) configuration;
Figure 4B is a side view of the vein filter of Figure 4A;
Figure 5 is a front view of the vein filter of Figure 4A;
Figure 6 is a side view of the vein filter of Figure 1 with the struts in the expanded configuration and the spacers in the collapsed configuration;
Figure 7 is a close up perspective view of the detail of Figure 6;
Figure 8 is a perspective view of a cranial end of a filter of the '429 application showing the retrieval hook of the filter;
Figure 9 is a view similar to Figure 8 except showing the cranial end of the filter of Figure 1, the spacers shown in the collapsed position;
Figure 9A is a perspective view of an alternate embodiment of the retrieval portion of the filter having an extended hook;
Figure 10 is a view similar to Figure 9, except showing a broken view of the spacers extending radially from the tubular portion;
Figure 11 is a perspective view of an alternate embodiment of a vein filter of the present invention having a single spacer loop, the filter and spacer shown in the expanded configuration;
Figure 12 is a front view of the filter of Figure 11;
Figures 13, 14, and 15 illustrate delivery and placement of the vessel filter of Figure 1 in the inferior vena cava wherein Figure 13 illustrates initial insertion of the delivery sheath through the femoral vein, Figure 14 illustrates the delivery sheath being advanced toward the inferior vena cava just below (upstream) the juncture of the renal arteries; and Figure 15 illustrates the filter in the expanded placement configuration in the inferior vena cava;
Figure 15A illustrates an initial step in removal of the filter from the inferior vena cava by a retrieval snare and catheter;
Figure 16 is a side view of an alternate embodiment of the filter of the present invention having spacers at different angles to the longitudinal axis of the filter, the spacers shown in the expanded position;
Figure 17 is a perspective view of an alternate embodiment of the filter of the present invention having a single spacer extending in a single plane, the spacers shown in the expanded position;
Figure 17A is a close up view of the area of detail of Figure 17;
Figure 18 is a perspective view of another alternate embodiment of a filter of the present invention having a single spacer extending in multiple planes;
Figure 19 is a perspective view of the cranial end of another alternate embodiment of a filter of the present invention having two spacers, the spacers shown in the expanded position;
Figure 19A is a view similar to Figure 19 except showing the spacers in the collapsed position;
Figure 20 is a perspective view of the cranial end of yet another alternate embodiment of a filter of the present invention having two spacers, the spacers shown in the expanded position;
Figure 20A is a view similar to Figure 20 except showing the spacers in the collapsed position;
Figure 21 is a perspective view of the cranial end of another alternate embodiment of a filter of the present invention showing the two spacers in the expanded position;
Figures 22A-22C illustrate another alternate embodiment of the cranial end of a filter of the present invention wherein Figure 22A is a perspective view of the cranial end in the collapsed configuration, Figure 22B is a side view in the collapsed configuration and Figure 22C is a perspective view in the expanded configuration.

### DETAILED DESCRIPTION OF PREFERRED EMBODIMENTS

Turning now to the drawings, wherein like reference numerals identify similar or like components throughout the several views, the vein filters of the present invention are described for placement within the inferior vena cava to capture blood clots or other particles which could otherwise pass to the lungs.

The filter is movable from a low profile collapsed configuration to facilitate insertion through the delivery sheath to a larger expanded placement configuration to enable atraumatic engagement with the vessel walls to secure (mount) the filter within the inferior vena cava. The filter is preferably substantially bell-shaped and preferably has a flared or mounting region (portion/section) and a filtering region (portion/section). The filtering region has inwardly directed struts, terminating in a converging region, thereby directing particles toward the central axis of the filter. By directing the particles to the center, they will be exposed to greater blood flow (since there is greater flow at the center than near the wall of the vessel) which improves dissolution of the particles. The filter increases in transverse dimension to form a flared region. The flare provides less contact area than a straight region, resulting in less tissue ingrowth to facilitate removal of the filter if desired. The flare also reduces the chance of vessel distortion if inserted into a curved vena cava. The filter also has spacers to space the cranial end of the filter from the vessel wall to facilitate removal.

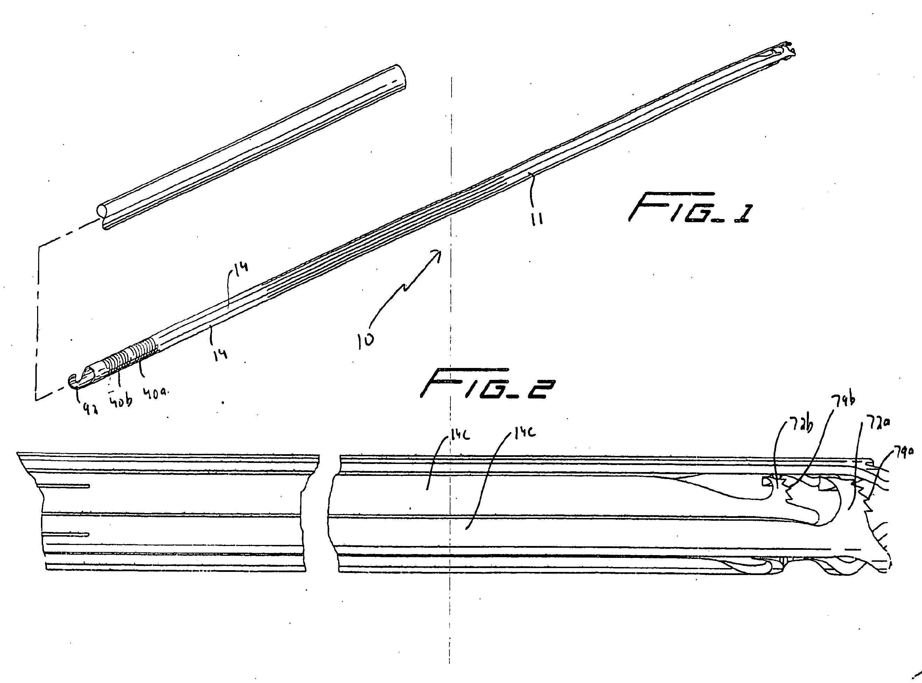

Turning now to details of the filter of the present invention and with initial reference to Figures 1 and 2, the filter is designated generally by reference numeral 10 and is shown in a collapsed configuration for delivery. Filter 10 is preferably formed from a single tube 11. In a preferred embodiment, the filter tube 11 is composed of shape memory material, such as Nitinol, a nickel titanium alloy, or elgiloy, however, other materials such as stainless steel are also contemplated. A plurality of cutouts are formed in the filter 10, preferably by laser cutting although other techniques are contemplated. In the illustrated embodiment, six elongated cutouts are formed, creating six strips or struts 14 of substantially uniform width separated by the cutouts.

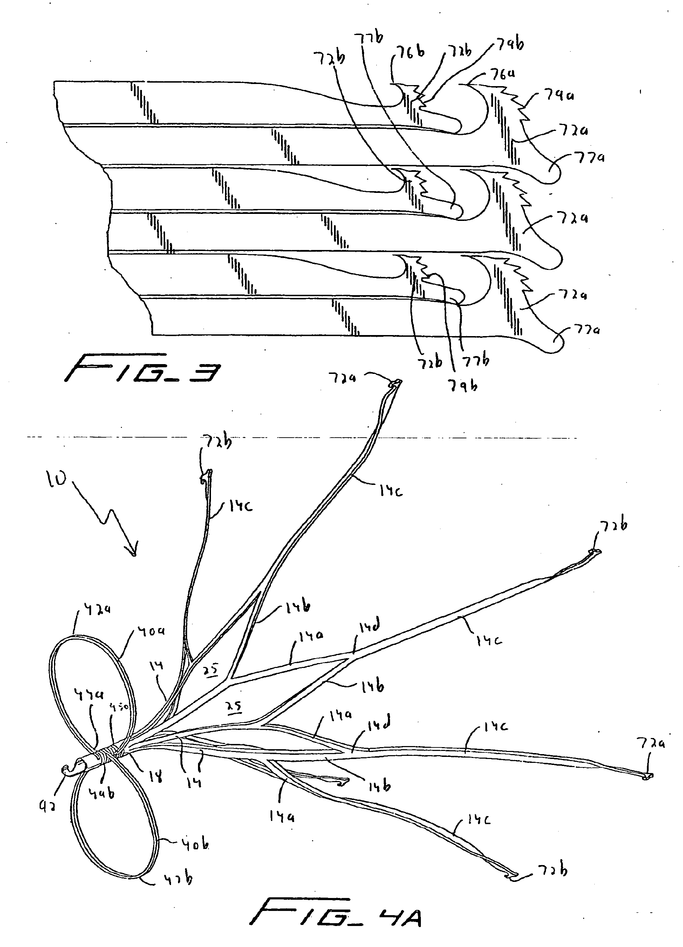

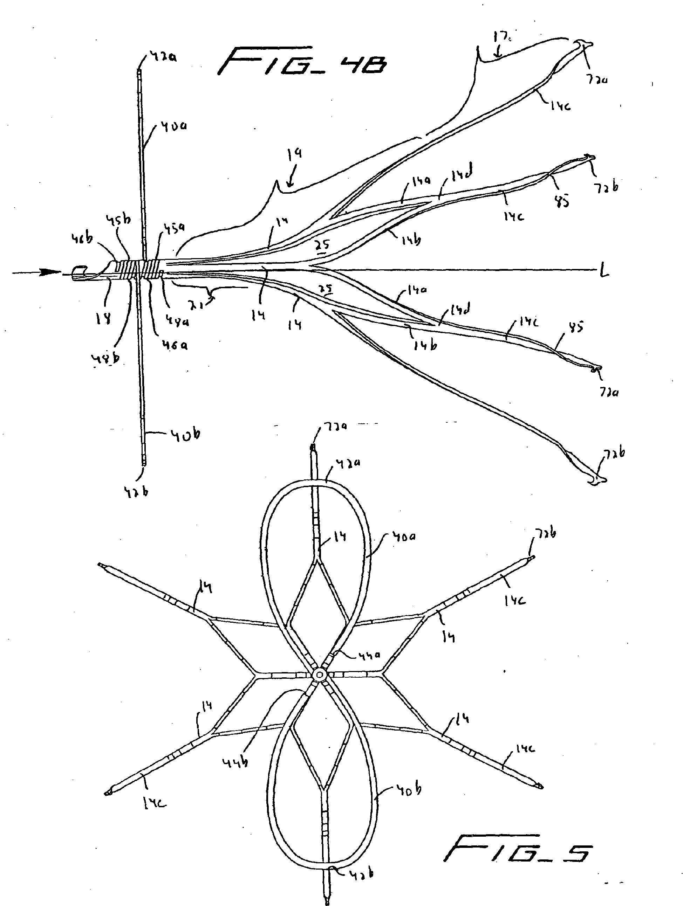

The collapsed configuration of filter 10 reduces the overall profile to facilitate delivery to the site. The diameter or transverse dimension of filter 10 in the collapsed configuration is preferably about 2mm and more preferably about 1.7mm. Other dimensions are also contemplated. The filter is thus preferably dimensioned for insertion through a 6 French delivery system and through a 6 French catheter. The diameter or transverse dimensions of the filter in the expanded placement configurations (e.g. Figs. 4A and 4B) is greater than the diameter or transverse dimension in the collapsed (delivery) configuration of Figure 1.

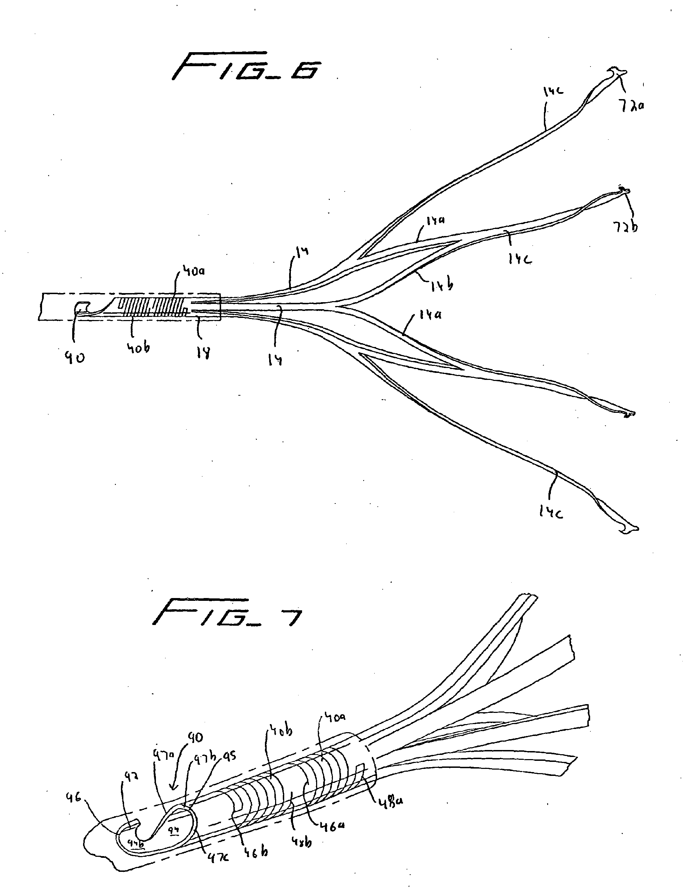

Figures 4-5 illustrate the expanded placement configuration of the filter 10. Figures 6 and 7 illustrate the expanded configuration of the struts with the spacers in the collapsed position (not exposed from the sheath) to help illustrate the invention, a configuration that would occur briefly. Filter 10 is generally bell-shaped in configuration. Filter 10 has a flared region 17 and a converging region 21 at the filtering section 19. The transverse dimension of the filter at the flared (or mounting/anchoring) region 17 is greater than the transverse dimension at filtering section 19. Diameters (or transverse dimensions) preferably range from about 18mm to about 32mm, depending on the internal diameter of the vessel wall as will be explained in more detail below. Other dimensions are also contemplated. The elongated struts 14 are spaced apart as shown and extend at an angle away from the longitudinal axis L of filter 10 in region 17 to provide a flare. Preferably, this angle or taper is about 8°, although other dimensions are contemplated. When expanded, the six struts 14, as shown, are preferably spaced approximately 60 degrees apart. It is also contemplated that a fewer or greater number of struts and spacing other than 60 degrees be provided.

Filtering section 19 extends from the flared region 17, and extends toward the central longitudinal axis L of the filter 10 and converges into tubular portion 18 at the cranial end of the filter.

The struts 14 of filter 10 terminate in hooks 72a, 72b which extend substantially perpendicular from the strut, achieved by torquing the struts at the region 85 so the hooks bend out of the plane. A first set of hooks 72a is larger than a second set of hooks 72b. Preferably when formed in a laser cut tube, hooks 72a are formed so that they occupy a region equivalent to the transverse dimension of two adjacent struts. Smaller hooks 72b are spaced axially with respect to each other and axially inwardly with respect to larger hooks 72a as in the filter hooks of the '429 application to minimize the collapsed profile (transverse dimension) of the filter when collapsed for insertion. The penetrating tips 76a, 76b of hooks 72a, 72b, respectively, penetrate the tissue to retain the filter, preferably temporarily, and point distally, toward the cranial (or distal) end of the filter.

Each of the hooks 72a, 72b has a series of teeth 79a, 79b, respectively to engage the vessel wall to provide additional retention to prevent movement of the filter in the caudal direction. In a preferred embodiment, the larger hooks 72a have four teeth and the smaller hooks 72b have three teeth, although a different number of teeth could be provided. A heel 77a, 77b, is provided which extends past (proximally or caudal of) the respective hook 72a, 72b to function as a stop to prevent the filter strut portions from going through the vessel wall. The angle of the heel 77b in the smaller hooks 72b is less than the angle in the larger hooks 72a to provide room for nesting of the hooks as shown in Figure 3. For clarity, not all of the hooks are fully labeled. Note this hook configuration with the teeth and/or heel can be utilized with the filters of the '429 application.

The six filter struts or strut portions 14 curve outwardly from tubular portion 18, extend radially therefrom and divide into two connecting filter struts or strut portions 14a, 14b (preferably of equal width, although differing dimensions are contemplated) that angle way from each other (in different directions) to extend to the connecting strut portion of an adjacent strut 14. Thus, connecting strut portion 14a of one strut 14 interconnects with the connecting strut portion 14b of an adjacent strut at joining region 14d. This forms closed geometric shapes 25, preferably substantially diamond shaped in configuration. For clarity, not all of the identical parts are labeled in the drawing.

In the illustrated embodiment, preferably six struts are provided forming twelve interconnecting struts, however a different number of struts and closed geometric shapes can be provided. Note that, although all six struts 14 are shown interconnected, it is also contemplated that fewer than all the struts can be interconnected. Also, the strut width can vary as described with respect to the filters disclosed in the '429 application.

After convergence of strut portions 14a, 14b at joining region 14d, it transitions into elongated mounting strut portions 14c which form flared mounting or anchoring region 17. The length of the strut portions 14c in the anchoring region 19 can vary, with increased/decreased length increasing the flexibility/rigidity of the struts. The thickness of the strut portions can also vary to affect flexibility/rigidity.

As in the other embodiments described in the '429 applications, terms such as interconnected, joined, etc., are used for ease of description, it being understood that preferably these portions are integral as they are preferably formed from a single tube. Also, mounting struts and filter struts used to describe the various embodiments disclosed herein can be considered as mounting strut "portions" or "sections" and filter strut "portions" or "sections" of the same struts if the filter is formed integrally, e.g. from a cut tube.

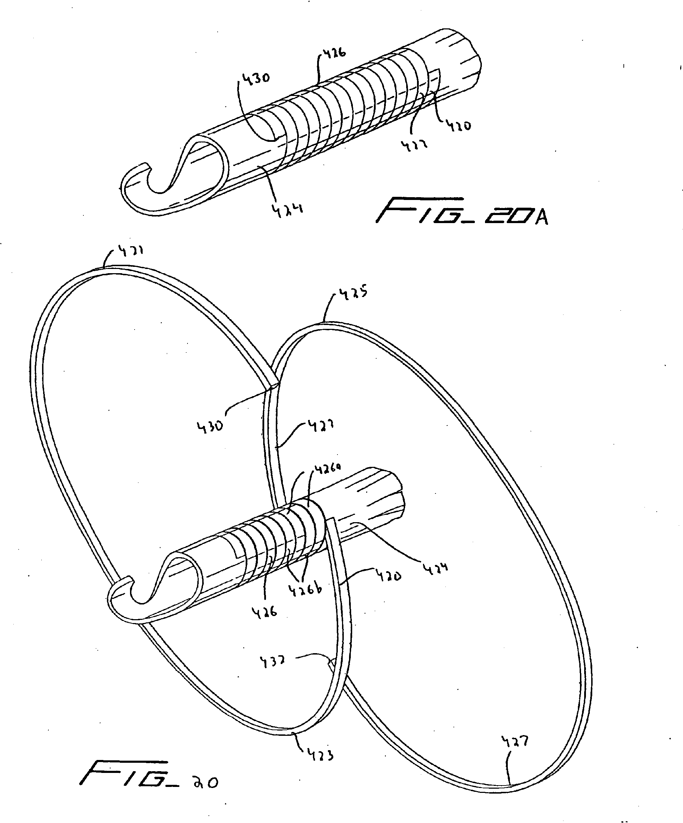

The tubular portion 18 is preferably in the form of a retrieval hook 92 as described with respect to the embodiment of Figure 20 in the '429 application. Other retrieval structure can also be utilized. Hook 92 is described in more detail below.

Two spiral cuts 45a, 45b are formed in the tube during manufacture, preferably by laser cutting, to enable two strips to be formed creating first and second spacers 40a, 40b for the filter. In the collapsed position, spacers 40a, 40b are in a substantially aligned position with respect to tubular portion 18, i.e. substantially flush with the tubular portion 18. Spacers 40a, 40b are maintained in this collapsed position during delivery to the surgical site. (see e.g. Figure 1). The spacers 40a, 40b have a shape memorized position forming loops as shown in Figure 4A. Thus, once exposed from the delivery sheath, the spacers 40a, 40b move from their collapsed position to their shape memory looped position of Figures 4A, 4B and 5. The surface 42a, 42b of the loop of each spacer 40a, 40b engages opposite sides (lying approximately 180° apart) of the vessel wall to maintain centering of the cranial end of the filter and to space tubular portion 18 and retrieval hook 92 away from the vessel wall. This spacing prevents tissue ingrowth around the hook, thereby making it easier to grasp and remove filter 10.

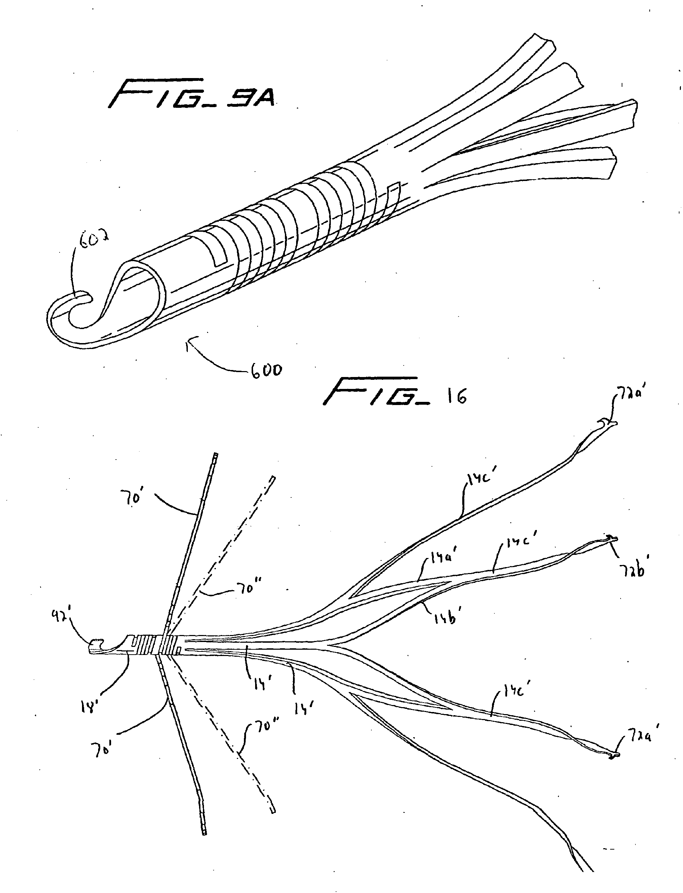

The loops of spacers 40a, 40b are open, somewhat oval shaped loops, terminate in ends 44a, 44b and lie in substantially alternate spiral planes. The first strip cut into tubular portion 18 unravels from a proximal end 48a of cutout 45a to a distal end 46a of cutout 45a to form spiral spacer 40a (see e.g. Figures 4B and 7). The second strip cut into tubular portion 18 unravels from a proximal end 48b of cutout 45b to a distal end 46b of cutout 45b to form spiral spacer 40b. In the Figure 4 embodiment, the spacer loops 40a, 40b lie in planes that are substantially perpendicular to the longitudinal axis L of the tubular portion 18 and filter 10. However, alternatively the spacer loops could lie in planes at angles other than 90 degrees and could lie in planes not parallel to each other. Examples of different angles of spacer loops with respect to the longitudinal axis of the tubular portion of the filter are shown by way of example in Figure 16. Two angled spacer loops (e.g. about 75 degrees) are designated by reference numeral 70' and smaller acute angled spacer loops, (e.g. about 45 degrees) are designated in phantom by reference numeral loop 70". The other components of the filter are identical to filter 10 and are designated with corresponding prime (') reference numerals.

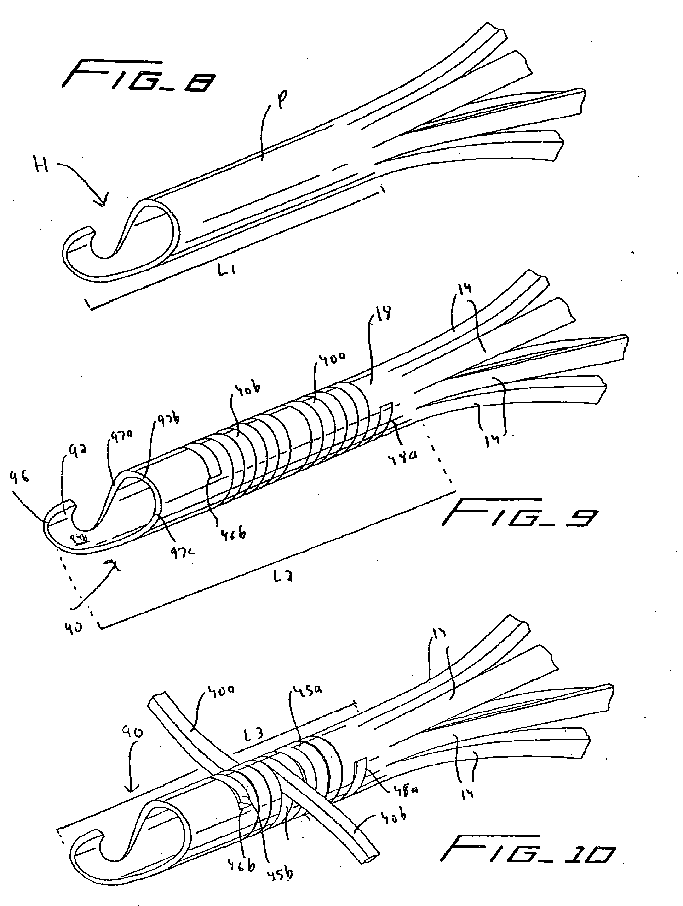

A comparison of Figures 8-10 illustrates that in the preferred embodiment, the length of tubular portion 18 remains substantially unchanged once the filter is implanted, even with the addition of the spacers. Figure 8 illustrates a cranial end of a filter of the '429 application showing the retrieval hook H of the filter. The tubular portion P has a length L 1 preferably ranging from about 2.54 mm (about .100 inches) to about 15.24 mm (about .600 inches), and preferably about 7.62 mm (about .300 inches). The tubular portion 18 of Figure 9, which is the embodiment of Figure 1, has a length L2, preferably ranging from about 12.7 mm (about .500 inches) to about 43.18 mm (about 1.700 inches), and preferably about 22.38 mm (about .881 inches) which is greater than length L1 due to the space needed to create the spiral spacers 40a, 40b. However, once the spacers 40a, 40b move from their aligned position to their expanded position, the end of the tubular portion 18 contracts to close the gap created by the spiral cutouts to move to a length L3 which is preferably closed to length L1.

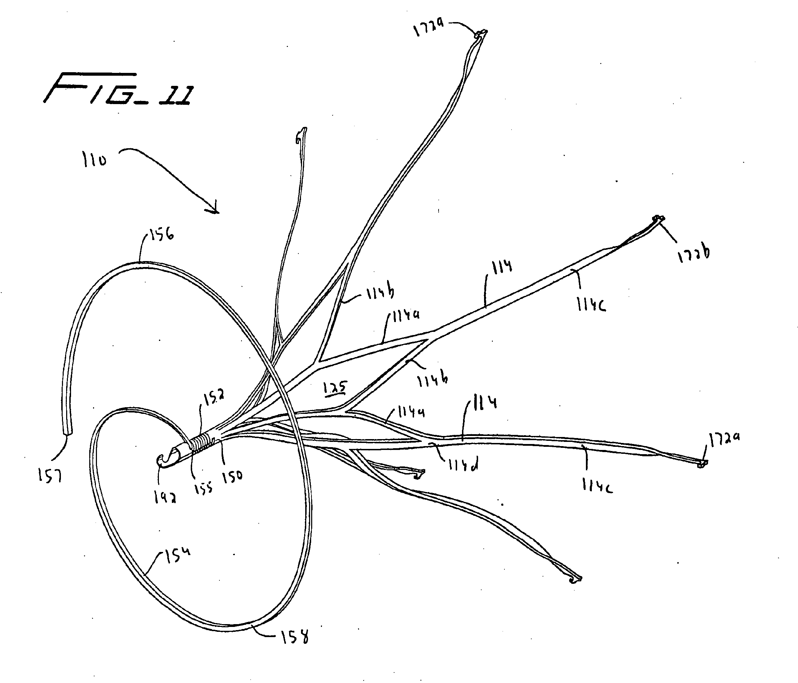

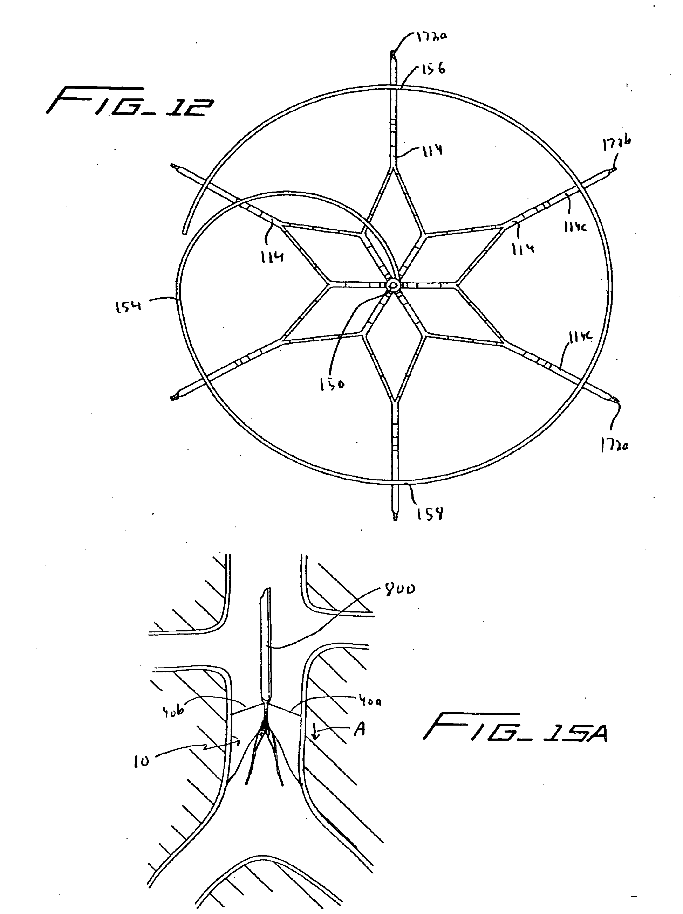

Figures 11 and 12 illustrate an alternate embodiment of the filter, designated by reference numeral 110. Filter 110 is identical to filter 10, except for the tubular portion and spacer, and therefore has been labeled with numerals in the "100" series corresponding to the double digit numbering of filter 10. Thus, filter 110 has struts 114, interconnecting struts 114a, 114b, hooks 172a, 172b, etc. and therefore for brevity these parts will not again be described.

Tubular portion 150 of filter 110 has a hook 192 identical to hook 92 of Figure 1. However, tubular portion 150 has a single spiral cutout 152, preferably formed by laser cutting, which forms a spiral spacer 154. The spiral spacer 154 has a shape memory position of that shown in Figures 11 and 12, extending radially from the tubular portion 150. When exposed from the sheath it unravels to move from a collapsed position substantially flush with the tubular portion 150 to its shape memory position forming an open loop starting at end 155 and wrapping over 360 degrees, terminating at edge 157. In this manner loop portions 156 and 158 are 180 degrees apart and the circular loop surfaces contact the inner wall of the vessel. It is also contemplated that the spacer can wrap a smaller or greater distance (degrees) than that shown and be oval or shapes other than circular. The loop can lie in a single plane or in multiple planes.

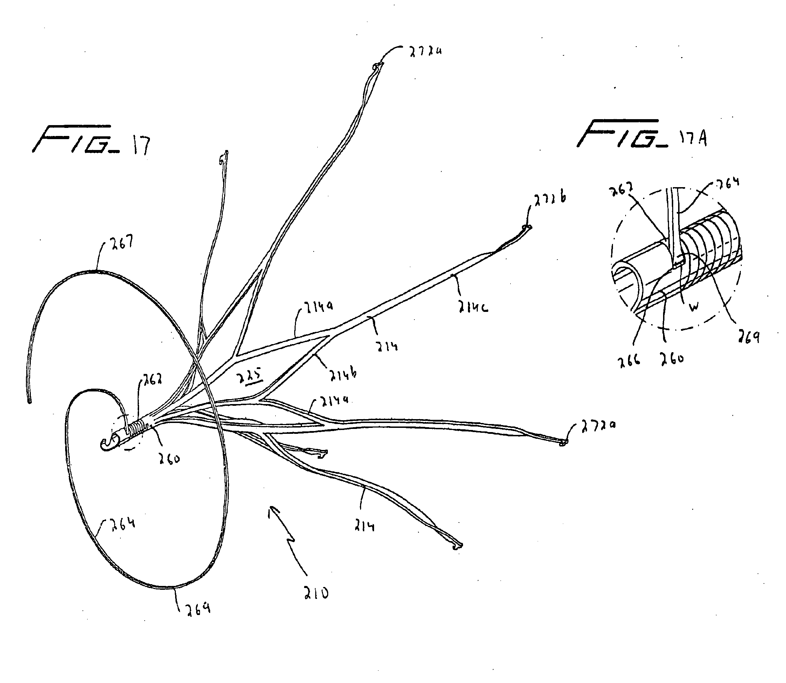

Figure 17 discloses an alternate embodiment of the filter which is identical to the filter shown in Figure 11 except for the spiral spacer 264. The filter 210 is labeled with reference numerals in the "200 series" corresponding to the "100 series" labeled parts of the Figure 11 embodiment and therefore has struts 214, hooks 272a, 272b, etc. The tubular portion and spacer, being different, have non-correlating reference numerals. More specifically, tubular portion 260 has a spiral cutout 262 to form a spacer 264. The distal (cranial) terminal end 266 of the cutout 262 has an increased width to form a spacer end 269 of increased width "w", shown in Figure 17 A. This provides increased support for the spacer as it reduces stress at that part. Spiral spacer 264 loops around tubular portion 260 in a similar manner as spacer 154 of Figure 11, preferably wrapping over 360 degrees, although other degrees are contemplated. Opposed looped ends 267 and 269 are about 180° apart and the outer surfaces contact opposing sides of the vessel wall. As with spacers 154, the outer surfaces along the loop contact the vessel wall due to the circular configuration of the spacer 264.

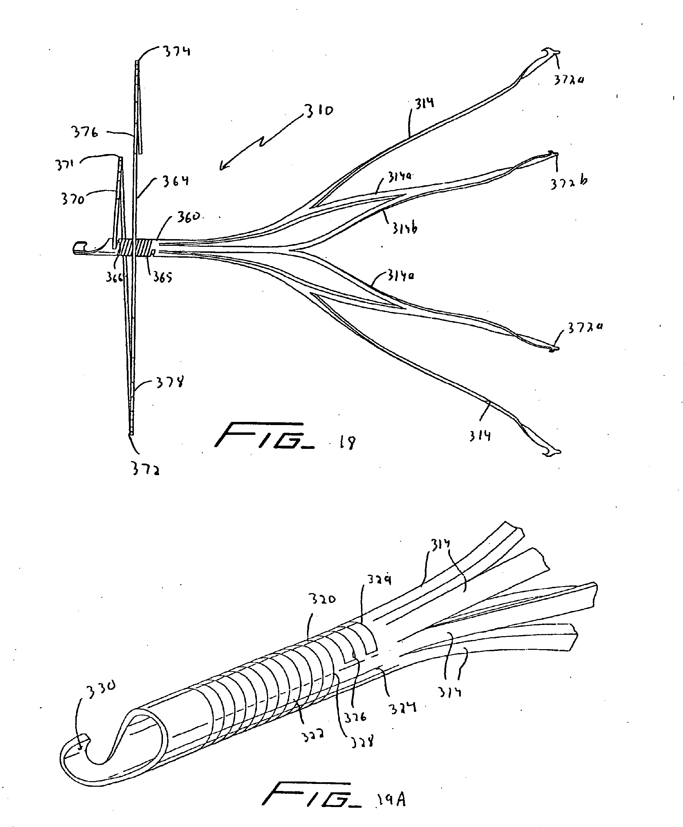

In the embodiment of Figure 18, the spacer 364 of filter 310 wraps around the tubular portion 360 in different planes, as opposed to the single plane of the embodiment of Figures 11 and 17. More specifically, in the expanded position, spacer 364 emerges from the distal (cranial) end 366 of cutout 365 and wraps at an angle toward the caudal end of the filter. Thus, as seen, the first end 372 of loop portion 370 lies in a plane proximal of the plane containing end 371 of loop portion 370 and distal of the plane containing the end 374 of loop portion 376. In other words, loop portion 378 lies, as viewed axially, between loops 370, 376. The remaining portions of the filter are identical to filter 210 of Figure 17 and are labeled with corresponding parts in the "300" series.

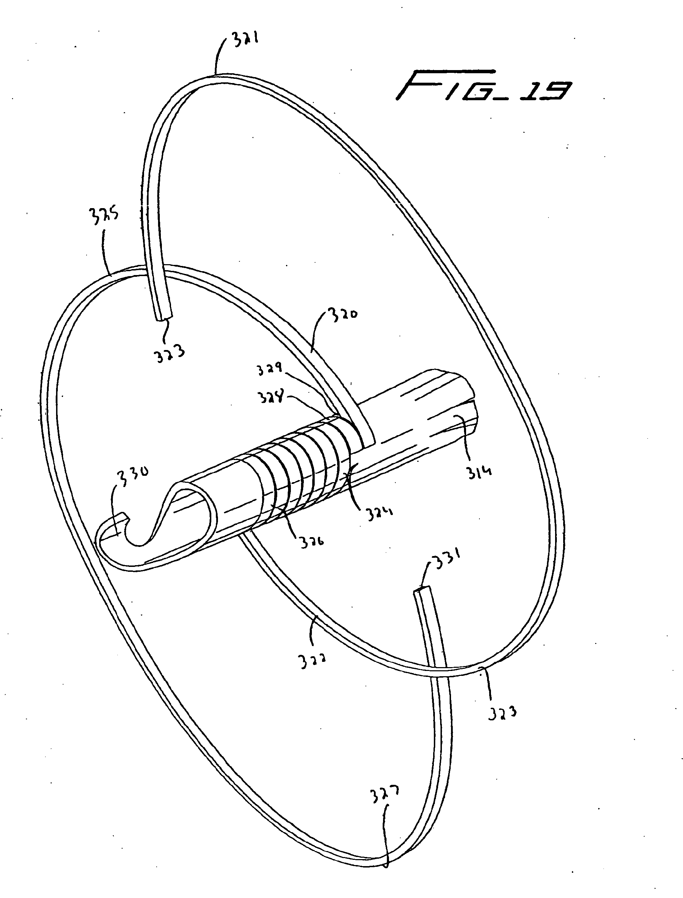

Figures 19 and 19A illustrate the cranial end of an alternate embodiment of the filter having two looped spacers extending radially from the tubular portion 324. In the collapsed position of Figure 19A, spacers 320, 322 are wrapped around tubular portion 324 so they are substantially flush with the wall of the tubular portion 324. The spacers 320, 322 are formed by two spiral cutouts 326, 328 formed in the wall of tubular portion 324. In the expanded position, spacer 320 emerges from the proximal (caudal) end 329 of cutout 328, extending in a substantially circular or spiral path around the tubular portion 324, preferably for about 300 degrees (although other degrees are contemplated), with surfaces 321, 323 about 180° apart contacting opposing surfaces of the vessel wall. Spacer loop 320 terminates at end 323 to form an open loop. Spacer 322 emerges from the distal (cranial) end of the cutout 326, wrapping around tubular portion 324 in the direction opposite of spacer 320. Similar to spacer 320, spacer 322 extends for about 300 degrees (although other degrees are contemplated), with opposing surfaces 325, 327 contacting opposite portions of the vessel wall. Spacer loop 322 terminates at end 331 to form an open loop. Hook 330 is preferably identical to hook 92 of the filter embodiment of Figure 1.

In the embodiment of Figure 20, open spacer loops 420 and 422 each start at a proximal end of cutout 426, with spacer 420 starting proximal of spacer 422. Cutout 426 has first cutout 426a and second cutout 426b, formed in an alternating pattern. Spacers 420, 422 lie in multiple planes, preferably wrap around tubular portion 424 in opposite directions, extending for about 300 degrees (although other degrees are contemplated) and have respective opposing surfaces 421, 423 and 425, 427, respectively for contacting opposing sides of the vessel wall. Spacers 420, 422 terminate in ends 430, 432.

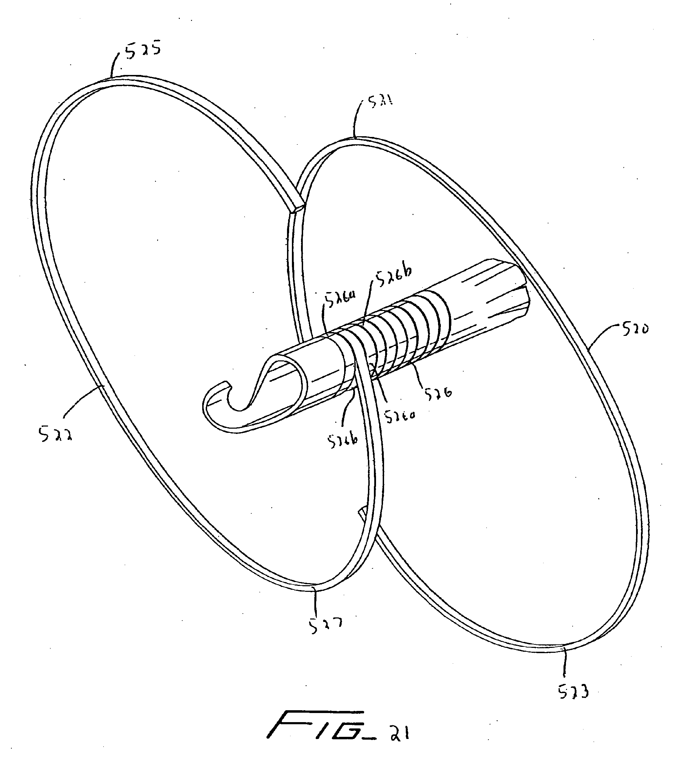

In the embodiment of Figure 21, open spacer loops 520, 522 are formed emerging from the distal end of cutouts 526a, 526b, with spacer 520 emerging distal of spacer 522. Spacers 520, 522 lie in different planes. Similar to loops 420, 422 of the embodiment of Figure 21, spacer 520 has opposing surfaces 521 and 523 and spacer 522 has opposing surfaces 525, 527. Loops 520, 522 lie in multiple planes.

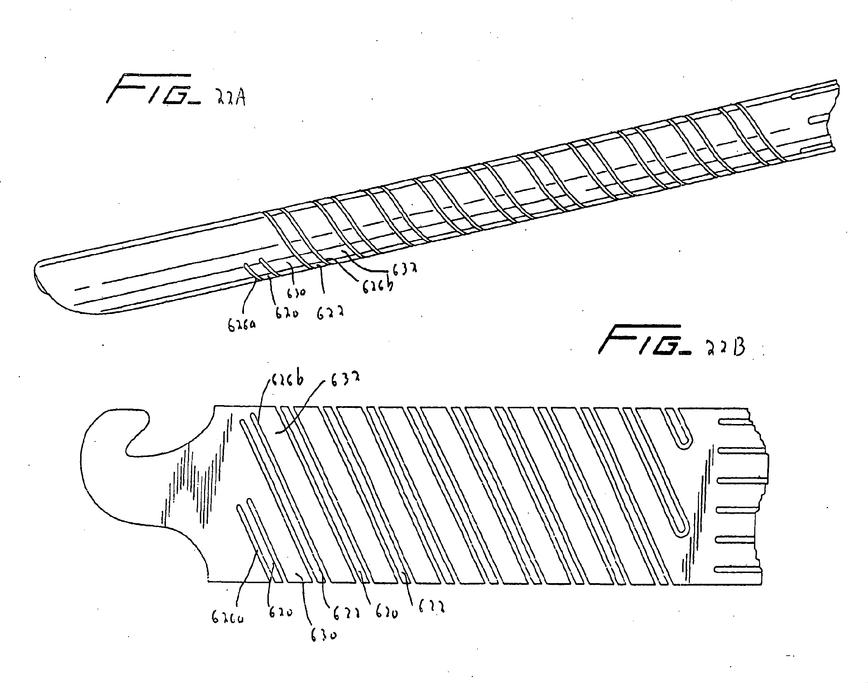

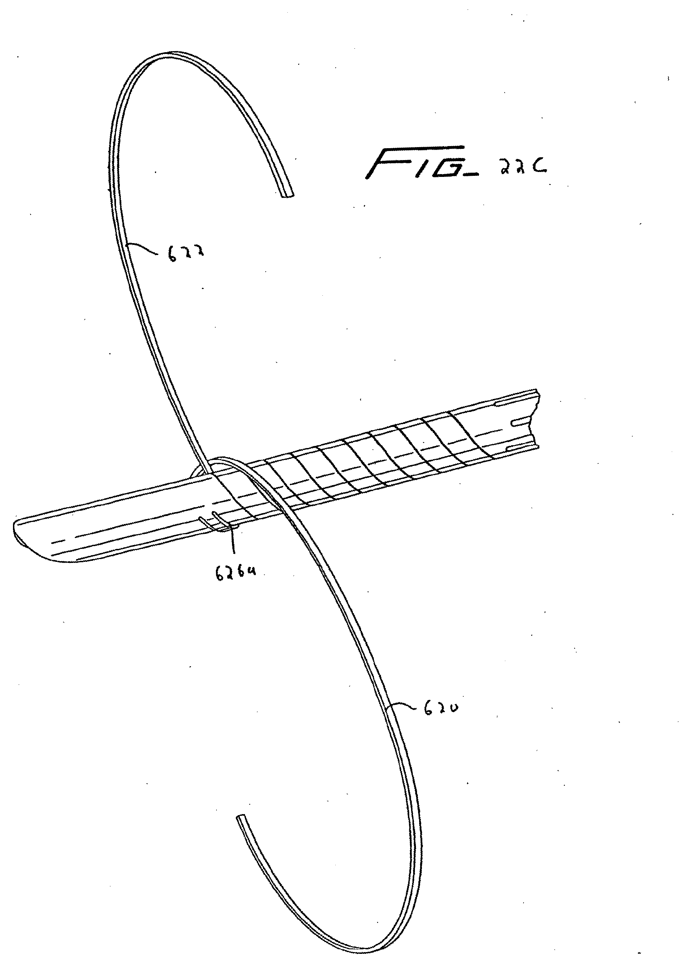

In the embodiment of Figure 22, open spacer loops 620, 622 extend from the distal end of cutout 626a, 626b. As shown, the cutouts are formed in an intertwined spiral fashion resulting in a spiral spacer when unraveled (expanded). The solid strip between cutout 626a is designated by reference numeral 630 and the solid strip between cutout 626b is designated by reference numeral 632.

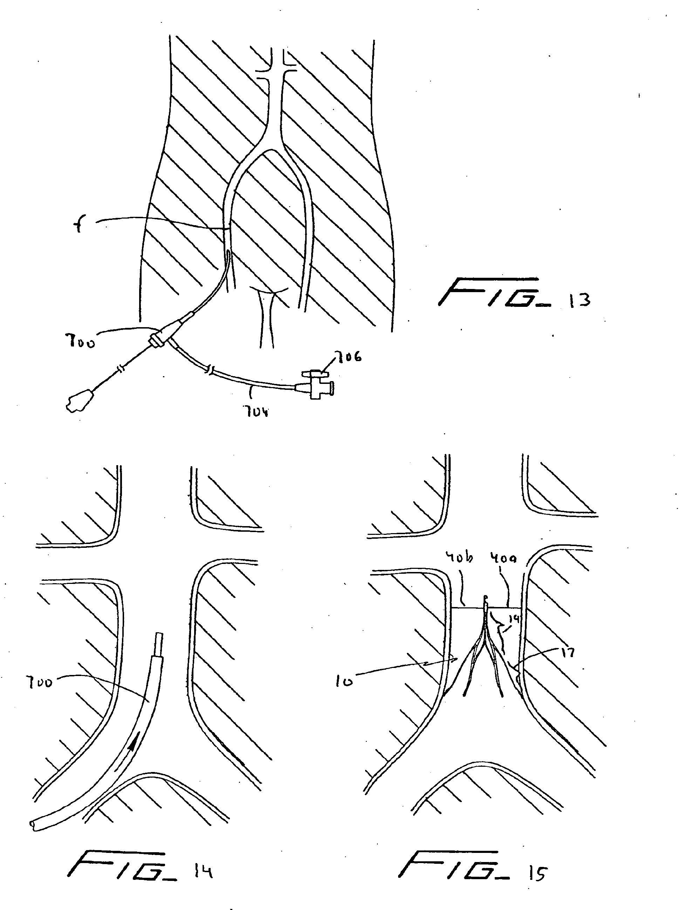

To enable movement between an expanded and collapsed configuration, the filter of the embodiments described herein, as noted above, is preferably made of shape memory metal material, such as Nitinol, a nickel titanium alloy, and preferably manufactured from a laser cut tube. To facilitate passage of the filter through the lumen of the delivery sheath 700 (shown in Figure 13 in conjunction with the method of insertion) and into the vessel, cold saline is injected into the delivery sheath or catheter 700 and around the filter in its collapsed position within the delivery sheath 700. This shape memory material characteristically exhibits rigidity in the austenitic state and more flexibility in the martensitic state. The cold saline maintains the temperature dependent filter in a relatively softer condition as it is in the martensitic state within the sheath. This facilitates the exit of filter from the sheath 700 as frictional contact between the filter and the inner surface of the sheath would otherwise occur if the filter was maintained in a rigid, i.e. austenitic, condition.

Once ejected from the delivery sheath or catheter 700, the filter is no longer cooled and is exposed to the warmer body temperature, which causes the filter to return towards its austenitic memorized configuration.

In the placement (expanded) configuration, the filter moves towards its memorized position and the extent it returns to its fully memorized position will be dependent on the size of the vessel in which the filter is inserted. (The larger the vessel, the closer the filter comes to returning to it's fully memorized position). The extent of movement of the spacer(s) to its fully memorized position could also be limited by the size of the vessel.

The filter can be inserted through the jugular vein in the neck of the patient or through the femoral vein in the leg of the patient or the arm. The filters can also be placed in the superior vena cava.

Figures 13-15 illustrate delivery and placement of the filter 10, by way of example, in the inferior vena cava. Delivery catheter or sheath 700 is inserted through the femoral vein "f" and advanced through the iliac arteries into the inferior vena cava. Delivery catheter 700 is withdrawn once the tip of the sheath is adjacent the structure so that withdrawal of the sheath would place the filter in the desired location of Figure 15. Tubing 704 and valve assembly 706 enable saline injection. Delivery catheter 700 is withdrawn to enable filter 10 to be warmed by body temperature to transition to the expanded placement configuration. The other filters described herein could be inserted in the same manner. Note it is implanted in the orientation such that filter section 19 is downstream of the flared section 17. This enables blood clots or other particles to be directed to the center of the filter section by the angled struts. Thus the direction of insertion, e.g. upstream or downstream direction, will determine how the filter is to be positioned in the delivery catheter.

The foregoing filters can be removed from access through the internal jugular or femoral vein. Various methods can be used to remove the filter such as those described in commonly assigned co-pending '429 application including for example, slotted hooks, graspers, etc.

A recess or cutout is preferably provided at the tubular end portion to form a hook portion 90, as shown for example in Figures 7 and 9, having a curved hook 92 at the proximalmost end to receive a snare or other device for removal as described in the filter of the '429 application.

This hook 92 is configured to receive a retrieval snare or other retrieval device. A portion of the wall of the hook 90 is cut out to expose the annular interior surface 94. This annular interior surface 94 extends from radiused region 95 to proximalmost edge 96. The interior surface 94, for ease of explanation, can be considered to have an interior surface at the radiused region 95 and an interior surface 94b at the hook 92. The interior surface 94b accommodates a portion of a tubular snare sheath. That is, the outer wall of the snare sheath (tube) can partially fit within the cut out region. This enhances removal as the snare pulls the filter hook into collinear arrangement with the sheath tube as described and shown in Figures 13H-13N of the '429 application. The radiused region 95, spaced axially (distal) from the hook 92, includes a radiused or curved edge defined by radiused side walls 97a, 97c and top wall 97b. The angled side walls 97a, 97c form camming surfaces to direct the hook 90 and filter into the retrieval sheath.

When the filter is grasped by the retrieval device and pulled distally to disengage from the vessel walls, the spacers flex inwardly. This is shown for example in Figure 15, wherein spacers 40a, 40b of filter 10 flex in the direction of the arrow as the filter is pulled into retrieval sheath 800.

It should be appreciated, that the hook can be formed in other ways to provide an interior annular surface to function in a similar manner as surface 94, i.e. to receive the snare tube. When the filter is pulled into the retrieval sheath it is collapsed for removal.

Figure 9A illustrates an alternate embodiment of the hook portion 600 having an elongated hook 602 curving inwardly. This provides increased hooking area for the retrieval snare.

To facilitate removal of the filter from the vessel, cold saline can be injected onto the implanted filter to change the temperature of the filter to move it to a relatively softer condition to facilitate the filter being drawn into the retrieval sheath. That is, injection of cold saline will cause the filter to approach its martensitic state, bringing the filter to a more flexible condition. The flexible condition facilitates the collapse and withdrawal of the filter into the retrieval sheath by decreasing the frictional contact between the filter and the inner surface of the retrieval sheath.

A delivery system which can be used for the filter of the present invention which includes a filter cartridge, is shown and described in the '429 application.

While the above description contains many specifics, those specifics should not be construed as limitations on the scope of the disclosure, but merely as exemplifications of preferred embodiments thereof. For example, the foregoing filters can be inserted in other regions of the body. Also, the foregoing filters can be made of materials other than shape memory material. Those skilled in the art will envision many other possible variations that are within the scope of the claims appended hereto.

## Claims

1. A vessel filter comprising a first region and a second region (17), the filter movable between a collapsed position for delivery to the vessel and a range of expanded positions for placement within the vessel, the first region having a filter portion (19) having a converging region (21) to direct particles toward the center of the filter, the first region including a plurality of spaced apart elongated struts (14, 114, 214) and a plurality of connecting struts (14a, 14b, 114a, 114b) extending at an angle from the elongated struts, the second region (17) being flared in the expanded positions to have a transverse dimension increasing toward a second end portion opposite the filter portion, the second region including a vessel engaging portion (72a, 72b, 172a, 172b, 272a, 272b) at the second end portion having a plurality of vessel engaging hooks, the region containing the hooks having a first transverse dimension, the first region having a spacer (40a, 40b, 70', 70", 154, 264, 364, 420, 422, 520, 522, 620, 622) extending radially with respect to a longitudinal axis of the filter, the spacer having a second transverse dimension, wherein in an unconstrained expanded position the second transverse dimension of the spacer is less than the first transverse dimension of the vessel engaging hook region to provide a lower profile at the spacer region, **characterized in that** the spacer (40a, 40b, 70', 70", 154, 264, 364, 420, 422, 520, 522, 620, 622) is formed from a spiral cutout (45a, 45b, 152, 262, 320, 322, 326, 328, 365, 426, 426a, 426b, 526a, 526b, 626a, 626b) in the first region of the filter.

2. A vessel filter as claimed in claim 1, **characterized in that** the spacer (40a, 40b, 420, 422, 520, 522, 620, 622) comprises two portions extending on opposite sides of the filter.

3. A vessel filter as claimed in claim 2, **characterized in that** the spacer (40a, 40b, 420, 422, 520, 522, 620, 622) comprises two looped portions.

4. A vessel filter as claimed in any preceding claim, **characterized in that** the spacer (40a, 40b, 70', 70", 154, 264, 364, 420, 422, 520, 522, 620, 622) is formed integrally with the filter and is formed of shape memory material.

5. A vessel filter as claimed in any preceding claim, **characterized in that** the vessel engaging portion (72a, 72b) includes vessel engaging hooks having a plurality of teeth (79a, 79b).

6. A vessel filter as claimed in claim 5, **characterized in that** the vessel engaging hooks (72a, 72b) include a heel (77a, 77b) extending past the hook.

7. A vessel filter as claimed in any preceding claim, **characterized in that** the filter is formed from a laser cut tube (11) and composed of shape memory material.

8. A vessel filter as claimed in any preceding claim, **characterized in that** the converging region (21) terminates in a tubular portion (18, 150, 260, 324, 360, 424) and each of the elongated struts (14, 114, 214) in the first region (19) extends outwardly from the tubular portion, the spacer (40a, 40b, 70', 70", 154, 264, 364, 420, 422, 520, 522, 620, 622) in the expanded position extending radially from the tubular portion.

9. A vessel filter as claimed in claim 8, **characterized in that** the spacer (40a, 40b, 70', 70", 154, 264, 364, 420, 422, 520, 522, 620, 622) is formed from a spiral (45a, 45b, 152, 262, 320, 322, 326, 328, 365, 426, 426a, 426b, 526a, 526b, 626a, 626b) cut in the tubular portion (18, 150, 260, 324, 360, 424).

10. A vessel filter as claimed in claim 8 or 9, **characterized in that** the spacer (40a, 40b, 70', 70", 154, 264, 364, 420, 422, 520, 522, 620, 622) has a looped shape memory position and during delivery has a collapsed position substantially flush with the tubular portion (18, 150, 260, 324, 360, 424).

11. A vessel filter as claimed in any preceding claim, **characterized in that** the first region further includes a retrieval region (90), the retrieval region including a hook (92, 192, 330) having a cutout exposing an internal annular surface (94), the annular surface dimensioned to receive a portion of a retrieval sheath (800).

## Patentansprüche

1. Gefäßfilter, der einen ersten Bereich und einen zweiten Bereich (17) umfasst, wobei der Filter zwischen einer zusammengeklappten Stellung der Abgabe an das Gefäß und einer Auswahl von ausgedehnten Stellungen zum Anordnen in dem Gefäß bewegt werden kann, der erste Bereich einen Filterabschnitt (19) mit einem zusammenlaufenden Bereich (21) aufweist, um Partikel zur Mitte des Filters zu leiten, der erste Bereich mehrere voneinander beabstandete längliche Streben (14, 114, 214) und mehrere Verbindungsstreben (14a, 14b, 114a, 114b), die sich in einem Winkel von den länglichen Streben erstrecken, beinhaltet, der zweite Bereich (17) in den ausgedehnten Stellungen aufgeweitet ist, um eine Querabmessung aufzuweisen, die zu einem zweiten Endabschnitt, der gegenüber dem Filterabschnitt liegt, zunimmt, der zweite Bereich am zweiten Endabschnitt einen Gefäßeingriffsabschnitt (72a, 72b, 172a, 172b, 272a, 272b) mit mehreren Gefäßeingriffshaken beinhaltet, wobei der Bereich, der die Haken beinhaltet, eine erste Querabmessung aufweist, der erste Bereich einen Abstandhalter (40a, 40b, 70', 70" , 154, 264, 364, 420, 422, 520, 522, 620, 622) aufweist, der sich radial in Bezug auf eine Längsachse des Filters erstreckt, wobei der Abstandhalter eine zweite Querabmessung aufweist, wobei die zweite Querabmessung des Abstandhalters in einer uneingeschränkten ausgedehnten Stellung kleiner als die erste Querabmessung des Gefäßeingriffshakenbereichs ist, um an dem Abstandhalterbereich ein niedrigeres Profil bereitzustellen, **dadurch gekennzeichnet, dass** der Abstandhalter (40a, 40b, 70', 70", 154, 264, 364, 420, 422, 520, 522, 620, 622) von einem spiralförmigen Ausschnitt (45a, 45b, 152, 262, 320, 322, 326, 328, 365, 426, 426a, 426b, 526a, 526b, 626a, 626b) in dem ersten Bereich des Filters gebildet wird.

2. Gefäßfilter nach Anspruch 1, **dadurch gekennzeichnet, dass** der Abstandhalter (40a, 40b, 420, 422, 520, 522, 620, 622) zwei Abschnitte umfasst, die sich auf gegenüber liegenden Seiten des Filters erstrecken.

3. Gefäßfilter nach Anspruch 2, **dadurch gekennzeichnet, dass** der Abstandhalter (40a, 40b, 420, 422, 520, 522, 620, 622) zwei schleifenförmige Abschnitte umfasst.

4. Gefäßfilter nach einem der vorhergehenden Ansprüche, **dadurch gekennzeichnet, dass** der Abstandhalter (40a, 40b, 70', 70", 154, 264, 364, 420, 422, 520, 522, 620, 622) einstückig mit dem Abstandhalter ausgebildet ist und aus Formgedächtnismaterial hergestellt ist.

5. Gefäßfilter nach einem der vorhergehenden Ansprüche, **dadurch gekennzeichnet, dass** der Gefäßeingriffsabschnitt (72a, 72b) Gefäßeingriffshaken mit mehreren Zähnen (79a, 79b) beinhaltet.

6. Gefäßfilter nach Anspruch 5, **dadurch gekennzeichnet, dass** die Gefäßeingriffshaken (72a, 72b) einen Sporn (77a, 77b) beinhalten, der sich an dem Haken vorbei erstreckt.

7. Gefäßfilter nach einem der vorhergehenden Ansprüche, **dadurch gekennzeichnet, dass** der Filter aus einem mit einem Laser geschnittenen Rohr (11) gebildet wird und sich aus Formgedächtnismaterial zusammensetzt.

8. Gefäßfilter nach einem der vorhergehenden Ansprüche, **dadurch gekennzeichnet, dass** der zusammenlaufende Bereich (21) in einem rohrförmigen Abschnitt (18, 150, 260, 324, 360, 424) endet und jede der länglichen Streben (14, 114, 214) in dem ersten Bereich (19) sich von dem rohrförmigen Abschnitt nach außen erstreckt, wobei der Abstandhalter (40a, 40b, 70', 70", 154, 264, 364, 420, 422, 520, 522, 620, 622) sich in der ausgedehnten Stellung radial von dem rohrförmigen Abschnitt erstreckt.

9. Gefäßfilter nach Anspruch 8, **dadurch gekennzeichnet, dass** der Abstandhalter (40a, 40b, 70', 70", 154, 264, 364, 420, 422, 520, 522, 620, 622) von einem spiralförmigen (45a, 45b, 152, 262, 320, 322, 326, 328, 365, 426, 426a, 426b, 526a, 526b, 626a, 626b) Schnitt in dem rohrförmigen Abschnitt (18, 150, 260, 324, 360, 424) gebildet wird.

10. Gefäßfilter nach Anspruch 8 oder 9, **dadurch gekennzeichnet, dass** der Abstandhalter (40a, 40b, 70', 70", 154, 264, 364, 420, 422, 520, 522, 620, 622) eine schleifenförmige Formgedächtnisstellung aufweist und während der Abgabe eine zusammengeklappte Stellung aufweiset, die im Wesentlichen mit dem rohrförmigen Abschnitt (18, 150, 260, 324, 360, 424) bündig ist.

11. Gefäßfilter nach einem der vorhergehenden Ansprüche, **dadurch gekennzeichnet, dass** der erste Bereich weiterhin einen Rückerlangungsbereich (90) beinhaltet, wobei der Rückerlangungsbereich einen Haken (92, 192, 330) mit einem Ausschnitt beinhaltet, der eine ringförmige Innenfläche (94) freilegt, wobei die ringförmige Fläche derart bemessen ist, dass sie einen Abschnitt einer Rückerlangungshülse (800) aufnimmt.

## Revendications

1. Filtre de vaisseau comprenant une première région et une seconde région (17), le filtre étant mobile entre une position repliée pour la livraison au vaisseau et une gamme de positions dilatées pour le placement au sein du vaisseau, la première région ayant une portion de filtre (19) ayant une région convergente (21) pour diriger des particules vers le centre du filtre, la première région incluant une pluralité d'entretoises allongées espacées (14, 114, 214) et une pluralité d'entretoises de connexion (14a, 14b, 114a, 114b) s'étendant à un angle depuis les entretoises allongées, la seconde région (17) étant évasée dans les positions dilatées pour avoir une dimension transversale augmentant vers une seconde portion d'extrémité opposée à la portion de filtre, la seconde région incluant une portion d'engagement de vaisseau (72a, 72b, 172a, 172b, 272a, 272b) au niveau de la seconde portion d'extrémité ayant une pluralité de crochets d'engagement de vaisseau, la région contenant les crochets ayant une première dimension transversale, la première région ayant un écarteur (40a, 40b, 70', 70", 154, 264, 364, 420, 422, 520, 522, 620, 622) s'étendant radialement par rapport à un axe longitudinal du filtre, l'écarteur ayant une seconde dimension transversale, dans lequel, dans une position dilatée non serrée, la seconde dimension transversale de l'écarteur est inférieure à la première dimension transversale de la région de crochet d'engagement de vaisseau pour fournir un profil inférieur au niveau de la région d'écarteur, **caractérisé en ce que** l'écarteur (40a, 40b, 70', 70", 154, 264, 364, 420, 422, 520, 522, 620, 622) est formé à partir d'une découpe en spirale (45a, 45b, 152, 262, 320, 322, 326, 328, 365, 426, 426a, 426b, 526a, 526b, 626a, 626b) dans la première région du filtre.

2. Filtre de vaisseau selon la revendication 1, **caractérisé en ce que** l'écarteur (40a, 40b, 420, 422, 520, 522, 620, 622) comprend deux portions s'étendant sur des côtés opposés du filtre.

3. Filtre de vaisseau selon la revendication 2, **caractérisé en ce que** l'écarteur (40a, 40b, 420, 422, 520, 522, 620, 622) comprend deux portions en boucle.

4. Filtre de vaisseau selon l'une quelconque des revendications précédentes, **caractérisé en ce que** l'écarteur (40a, 40b, 70', 70", 154, 264, 364, 420, 422, 520, 522, 620, 622) est formé de manière intégrale avec le filtre et est formé en un matériau à mémoire de forme.

5. Filtre de vaisseau selon l'une quelconque des revendications précédentes, **caractérisé en ce que** la portion d'engagement de vaisseau (72a, 72b) inclut des crochets d'engagement de vaisseau ayant une pluralité de dents (79a, 79b).

6. Filtre de vaisseau selon la revendication 5, **caractérisé en ce que** les crochets d'engagement de vaisseau (72a, 72b) incluent un talon (77a, 77b) s'étendant au-delà du crochet.

7. Filtre de vaisseau selon l'une quelconque des revendications précédentes, **caractérisé en ce que** le filtre est formé à partir d'un tube découpé au laser (11) et composé d'un matériau à mémoire de forme.

8. Filtre de vaisseau selon l'une quelconque des revendications précédentes, **caractérisé en ce que** la région convergente (21) se termine dans une portion tubulaire (18, 150, 260, 324, 360, 424) et chacune des entretoises allongées (14, 114, 214) dans la première région (19) s'étend vers l'extérieur depuis la portion tubulaire, l'écarteur (40a, 40b, 70', 70", 154, 264, 364, 420, 422, 520, 522, 620, 622) dans la position dilatée s'étendant radialement depuis la portion tubulaire.

9. Filtre de vaisseau selon la revendication 8, **caractérisé en ce que** l'écarteur (40a, 40b, 70', 70", 154, 264, 364, 420, 422, 520, 522, 620, 622) est formé à partir d'une spirale (45a, 45b, 152, 262, 320, 322, 326, 328, 365, 426, 426a, 426b, 526a, 526b, 626a, 626b) découpée dans la portion tubulaire (18, 150, 260, 324, 360, 424).

10. Filtre de vaisseau selon la revendication 8 ou 9, **caractérisé en ce que** l'écarteur (40a, 40b, 70', 70", 154, 264, 364, 420, 422, 520, 522, 620, 622) possède une position à mémoire de forme en boucle et possède une position repliée essentiellement à fleur avec la portion tubulaire (18, 150, 260, 324, 360, 424) au cours de la livraison.

11. Filtre de vaisseau selon l'une quelconque des revendications précédentes, **caractérisé en ce que** la première région inclut en outre une région de récupération (90), la région de récupération incluant un crochet (90, 192, 330) ayant une découpe exposant une surface annulaire interne (94), la surface annulaire étant dimensionnée pour recevoir une portion d'un manchon de récupération (800).
